# EUROPEAN PATENT SPECIFICATION

(11) **EP 1 478 242 B1**
(45) Date of publication and mention of the grant of the patent: **30.04.2014**
(21) Application number: 03742552.7
(22) Date of filing: 18.02.2003
(51) Int. Cl.: A23K 1/00, A23L 1/03, A23K 1/18

(54) **PET FOOD COMPOSITION FOR SKIN PHOTOPROTECTION**
FUTTERMITTEL FÜR HAUSTIERE ZUM SCHUTZ DER HAUT GEGEN LICHT
COMPOSITION ALIMENTAIRE POUR ANIMAUX DE COMPAGNIE POUR LA PHOTOPROTECTION DE LA PEAU

(30) Priority: 21.02.2002 EP 02075702
(43) Date of publication of application: 24.11.2004
(73) Proprietor: SOCIETE DES PRODUITS NESTLE S.A., 1800 Vevey (CH)
(72) Inventor: BRETON, Lionel, F-78000 Versailles (FR); BUREAU-FRANZ, Isabelle, CH-1052 Le Mont-Sur-Lausanne (CH)
(74) Representative: Rupp, Christian
(86) International application number: PCT/EP2003/001687
(87) International publication number: WO 2003/070019

(56) References cited:
- EP-A- 0 286 033
- EP-A- 0 904 784
- WO-A-01/17365
- DE-A- 3 536 342
- FR-A- 2 757 769
- US-A- 5 702 719
- US-A- 5 968 569
- US-A1- 2002 039 606
- US-B1- 6 270 811
- DATABASE WPI Section Ch, Week 200232 Derwent Publications Ltd., London, GB; Class B04, AN 2002-278805 XP002207609 & RU 2 178 975 C (BORTS M S), 10 February 2002 (2002-02-10)

## Description

### Field of the invention

The present invention relates to pet food composition, for the photoprotection of the skin, whether before, during and/or after exposure to UV radiation, and to the use of same for preventing and/or attenuating the damage caused by such UV irradiation. It also relates to a method to improve the photoprotection of the skin.

### Background of the Invention

The continuous decrease of the atmosphere's ozone layer with the concurrent increase of ultraviolet radiation reaching the planet's surface has attracted a great deal of interest in its potential consequence on human health.

Indeed, it is known that light radiation of wavelengths of from 320 nm to 400 nm (UV-A) promotes tanning of the human epidermis; such radiation, however, is likely to cause damage to the skin, especially in the case of sensitive skin or skin which is continuously exposed to solar radiation. UV-A rays cause, in particular, a loss in the elasticity of the skin and the appearance of wrinkles, promoting a premature aging thereof. It is also known to this art that light rays having wavelengths of from 280 to 320 nm (UV-B) cause erythema and skin burning which can impair the natural development of a tan.

Although exposure to ultraviolet radiation is needed to produce vitamin D, growing evidence suggests that extensive exposure to sun-light, in particular to ultraviolet radiation, causes a variety of problems in the skin, including induction of certain skin cancers and induction of accelerated skin ageing.

In addition to these established health concerns, research has also provied evidence suggesting that exposure to ultraviolet radiation may negatively affect a variety of immune responses in living beings both locally, within the UV-irradiated skin, and also systemically, i.e. at sites distant from the irradiated skin.

It is thus necessary, in order to maintain suitable skin quality after exposure to UV radiation, to prepare or treat the skin before the exposure, to protect it during the exposure and even to alleviate the detrimental effects of ultraviolet radiation on the skin, prevent the development of erythema, edema and/or flaking or scaling (hyperkeratosis) of the skin.

There is thus a need in the art for an orally administrable composition which is capable to improve and/or reinforce the photoprotective function of the skin of pets.

### Summary of the Invention

Accordingly, in a first aspect the present invention aims to provide an ingestable composition for the photoprotection of the skin which comprises i) at least one probiotic lactic acid bacteria or a culture supernatant thereof, ii) at least one yeast, and iii) carotenoid.

Food compositions comprising probiotic lactic acid bacteria are known. However, the photoprotective effects of such compositions has not previously been recognized.

Pet food composition comprising probiotic micro-organisms such as yeasts moulds and lactic acid bacteria are disclosed in US 5,9678,569. Typically each composition comprises a single probiotic micro-organism in combination with a carrier. The compositions are disclosed to have a beneficial effect on the gastro-intestinal tract of the pet.

RU 2178975 also discloses food compositions useful for improving the function of the gastro-intestinal tract. The compositions typically comprise a lactic acid bacteria and an anti-oxidant such as vitamin A.

DE 3536342 discloses a pharmaceutical composition comprising a lactic acid bacteria and a variety of additives such as yeast for the treatment of skin disorders.

EP 0 904 784 discloses a food supplement useful for treatment of disorders of the gastro-intestinal tract comprising a probiotic lactic acid bacteria and a carotenoid. The compositions may also include prebiotic compounds as well as substances that inhibit bacterial adhesion to the wall of the gastro-intestinal tract.

WO 01/17365 discloses methods and compositions for improving the condition of a pet's skin and coat. The method involves administering a composition a probiotic or a probiotic micro-organism or both, optionally together with a long chain fatty acid. The probiotic micro-organisms typically include yeasts, moulds, lactobacillus bacteria, bifido-bacteria and streptococcus bacteria amongst others.

The present invention further relates to the use of a photoprotecting effective amount of i) at least one prebiotic lactic acid bacteria or a culture supernatant thereof; ii) at least one yeast and iii) a carotenoid or derivative, for preparing an ingestable carrier for protecting the skin of pets against radiations such as ultraviolet and all related skin disorders, such as erythema, inflammation, sun burn, barrier function, photoageing, alteration of the immune system, for example.

In a last aspect, the invention relates to a method for improving the photoprotective function of the skin of pets, which comprises the step of orally administering to the pet a composition comprising a photoprotecting effective amount of i) at least one probiotic lactic acid bacteria or a culture supernatant thereof, and ii) at least one yeast, in an ingestible carrier.

The invention also provides a method of reducing the effects of ageing in a pet comprising the step of feeding a pet a pet food composition as described above.

The combination according to the present invention has a particular beneficial effect on skin protection and colouration of the skin, that helps to reduce the effects of ultraviolet-related stress on skin.

### Detailed Description of the Invention

Within the following description, "NCC" designates Nestlé Culture Collection (Nestlé Research Center, Vers-chez-les-Blanc, Lausanne, Switzerland). The term "photoprotection" is iused to describe attempt to block or reduce the adverse clinical, histological and immunological effects of solar radiation exposure on the skin.

Accordingly, the present invention provides an ingestable composition for use in the protection of the skin of pets against solar radiations and the attenuation or prevention of all related skin disorders of pets which comprises at least one probiotic lactic acid bacterium or a culture supernatant thereof and at least one yeast.

Indeed, it has now surprisingly and unexpectedly been determined that admixture of these very specific constituents elicits an enhanced effect or response in respect of the photoprotection of the skin.

Probiotics are non-pathogenic and non-toxigenic organisms, that survive passage through the stomach and small intestine. Upon continuous ingestion by the host they eventually may colonize the gut to a substantial extent thus competing with other potentially pathogenic bacteria for nutrients and/or attachment sites on the gastrointestinal wall and reducing their numbers and reducing or preventing infections. Until now a number of different probiotic micro-organisms have been found, which all are reported to exert their effect in the gut via the production of toxins, metabolic by-products, short chain fatty acids and the like.

It has now been shown that probiotics do also exert an effect in an individual's body at a location distant from the region in which they colonize it. And particularly, it has been surprinsingly found that a composition having a synergistic photoprotective effect on the skin may be obtained by combining into an ingestable carrier, a probiotic micro-organism and a yeast.

In a preferred embodiment, the probiotic to be included into the carrier is selected from the group consisting of lactic acid bacteria, in particular Lactobacilli, Bifidobacteria or Enterococci; and are more preferably *Lactobacillus johnsonii, Lactobacillus reuteri, Lactobacillus rhamnosus, Lactobacillus paracasei, Lactobacillus casei* or *Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum, Enterococcus faecium, Enterococcus sp.or* a mixture thereof.

According to a most preferred embodiment the strains *Lactobacillus johnsonii* NCC *533, Lactobacillus paracasei* NCC 2461, *Bifidobacterium adolescentis* NCC 251 and *Bifidobacterium longum* NCC 490 were deposited by way of an example, under the Budapest Treaty with the Institut Pasteur (28 rue du Docteur Roux, F-75024 Paris cédex 15) on 30.06.92, 12.01.99, 15.04.99 and 15.03.99, respectively and under the deposit number CNCM I-1225, CNCM I-2116, CNCM I-2168 and CNCM I-2170, respectively.

The strain of *Bifidobacterium lactis* (ATCC27536) provided by Hansen (Chr. Hansen A/S, 10-12 Boege Alle, P.O. Box 407, DK-2970 Hoersholm, Danemark) can also be used.

The probiotic microorganism according to the present invention may be included in a live form, semi-active or in desactivated form, e.g. as a lyophilized powder. Also culture supernatants of the microorganisms may be included in the products, optionally in concentrated form. It may also be included in an encapsulated form. When using a supernatant of a probiotic's culture the supernatant may be used as such or may be subjected to one or more purification steps prior to inclusion into the product, so as to concentrate or isolate the active ingredient (s) /metabolite (s). Method and techniques for purifying compounds and detecting the activity thereof in the fractions obtained are well known to the skilled person.

The probiotic lactic acid bacteria may be present in the carrier in an amount of at least 10⁵ cfu/ g of ingestable carrier, preferably from about 10⁵ to 10¹⁵ cfu/ g of ingestable carrier, and more preferably from 10⁷ to 10¹²cfu/ g of ingestable carrier.

It may be incorporated in dispersion form in a suitable vehicle such as water, organic solvents and fatty substances including oils, whether alone or in admixture.

The compositions according to the invention also comprise a yeast. In a preferred embodiment, the yeast is any food-grade yeast selected from the group consisting of Ascomycotina or Deuteromycotina . In a preferred embodiment, the yeast may be selected from the group consisting of *Debaryomyces, Kluyveromyces, Saccharomyces, Yarrowia, Zygosaccharomyces, Candida and Rhaodutorula,* and more preferably *Saccharomyces caerevisae* (baker's yeast).

Such yeast may be used in the form of dried or lyophilized extracts. It may be present in the carrier in an amount of at least 10⁵ cfu/ g of ingestable carrier, preferably from about 10⁵ to 10¹⁵ cfu/ g of ingestable carrier, and more preferably from 10⁷ to 10¹²cfu/ g of ingestable carrier, said amount depending on the nature and activity of the particular yeast.

The carotenoid may be a carotenoid with or without provitamin A activity. It may be β-carotene, γ-carotène, α-carotene, lycopene, zeaxanthine and luteine, or a mixture thereof. The carotenoid may be from synthetic or natural origin or contained in a natural extract. When the carotenoid is from natural origin, it is preferably obtained from plant material, in which the plant is grown in-vivo or in-vitro. Method for extracting the carotenoids are well known in the art. The carotenoid may be present in the carrier in an amount of from 10⁻¹²% to 20% by weight and preferably from 0,00001 mg to 50 mg/day and more preferably from 0.001mg to 30mg/day.

A mixture of a plurality of lactic acid bacteria, yeast and/or carotenoids may also be used.

The carrier may be any pet food or pharmaceutical product, or a nutritional supplement or a treat, wherein the probiotic microorganism, the yeast and/or carotenoid may be included. Methods for preparing the carrier are common knowledge.

The said composition may be administered to the pet as a supplement to its normal diet or as a component of a nutritionally complete pet food. It may also be a pharmaceutical composition.

The nutritionally complete pet food composition according to the invention may be in powdered, dried form, a treat or a wet, chilled or shelf stable pet food product. These pet foods may be produced by ways known in the art. Apart from the plant or plant extract, these pet foods may include any one or more of a starch source, a protein source and a lipid source.

Suitable starch sources are, for example, grains and legumes such as corn, rice, wheat, barley, oats, soy, and mixtures of these. Suitable protein sources may be selected from any suitable animal or vegetable protein source; for example meat and meal, poultry meal, fish meal, soy protein concentrates, milk proteins, gluten, and the like. For elderly animals, it is preferred for the protein source to contain a high quality protein. Suitable lipid sources include meats, animal fats and vegetable fats. The choice of the starch, protein and lipid sources will be largely determined by the nutritional needs of the animal, palatability considerations, and the type of product applied. For elderly pets, the pet food preferably contains proportionally less fat than pet foods for younger pets. Furthermore, the starch sources may include one or more of rice, barley, wheat and corn.

The pet food may optionally also contain a prebiotic or another active agent, for example a long chain fatty acid. The amount of prebiotic in the pet food is preferably less than 10% by weight. For example, the prebiotic may comprise about 0.1 % to about 5% by weight of the pet food. For pet foods which use chicory as the source of the prebiotic, the chicory may be included to comprise about 0.5% to about 10% by weight of the feed mixture; more preferably about 1% to about 5% by weight.

Suitable long chain fatty acids include linoleic acid, alpha-linolenic acid, gamma linolenic acid, eicosapentanoic acid, and docosahexanoic acid. Fish oils are a suitable source of eicosapentanoic acids and docosahexanoic acid. Borage oil, blackcurrant seed oil and evening primrose oil are suitable sources of gamma linoleic acid. Safflower oils, sunflower oils, corn oils and soybean oils are suitable sources of linoleic acid.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

For dried pet food a suitable process is extrusion cooking, although baking and other suitable processes may be used. When extrusion cooked, the dried pet food is usually provided in the form of a kibble. If a prebiotic is used, the prebiotic may be admixed with the other ingredients of the dried pet food prior to processing. A suitable process is described in European patent application No 0850569. If a probiotic microorganism is used, the organism is preferably coated onto or filled into the dried pet food. A suitable process is described in European patent application No 0862863.

For wet food, the processes described in US patents 4,781,939 and 5,132,137 may be used to produce simulated meat products. Other procedures for producing chunk type products may also be used; for example cooking in a steam oven. Alternatively, loaf type products may be produced by emulsifying a suitable meat material to produce a meat emulsion, adding a suitable gelling agent, and heating the meat emulsion prior to filling into cans or other containers.

In another aspect, the invention provides the use of a composition comprising at least one probiotic lactic acid bacterium or a culture supernatant thereof and at least one yeast in the preparation of an ingestable composition for the protection of the skin of pets against solar radiations and the attenuation or prevention of all related skin disorders of pets.

The amount of the composition to be consumed by the individual will depend on the desirable effect. However, an amount of the composition to provide a daily amount of about 10⁵ to 10¹² organisms, which organism may be alive or dead, and 0.00001 to 50 mg of carotenoids would usually be adequate.

The composition is administered to an individual before or during the exposure to ultraviolet radiations, in particular exposure to sun. When the exposure period is foreseeable, it is desirable to start the consumption of the composition from 10 to 20 days before, and to prolong consumption during exposure.

In order to further illustrate the present invention and the advantages thereof, the following specific examples are given, it being understood that same are intended only as illustrative and in nowise limitative. In said examples to follow, as in the above description, all parts and percentages are given by weight, unless otherwise indicated.

### Examples

### Example 1 : Dry dog food

A feed mixture is made up of about 58% by weight of corn, about 5.5% by weight of corn gluten, about 22% by weight of chicken meal, 2,5% dried chicory, salts, vitamins and minerals making up the remainder.

The fed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets. At this point, a lyophilized powder of one strain of the following strains : CNCM I-1225, CNCM I-2116, CNCM I-2168, CNCM I-2170 or ATCC 27536 and lyophilized powder of *S.cerevissae* (so that the corresponding amount of each is about 1.0E+05-1.0E+12 cfu / day) can be added and mixed to the product. Furthermore, the same amount of bacteria can be sprayed on the cooled pellets after the pellets are dried at 50-60°C for some minutes.

This dry dog food helps to protect the skin's natural defenses of pets against the sun's harmful UV rays.

### Example 2: Canned pet food and supplement.

A mixture is prepared from 73 % of poultry carcass, pig lungs and beef liver (ground), 16 % of wheat flour, 2 % of dyes, vitamins, and inorganic salts. This mixture is emulsified at 12°C and extruded in the form of a pudding which is then cooked at a temperature of 90°C. It is cooled to 30°C and cut in chunks. 45 % of these chunks are mixed with 55 % of a sauce prepared from 98 % of water, 1 % of dye, and 1 % of guar gum. Tinplate cans are filled and sterilized at 125°C for 40 min. As a probiotic supplement to be mixed with the pet-food before serving, additional packaging (e.g. sachet) with one of the following strains: CNCM I-1225, CNCM I-2116, CNCM I-2168, CNCM I-2170 or ATCC 27536, and β-carotene and lyophilized *S.cerevissae.* The corresponding amount for the pet is about 10⁵-10¹² cfu / day microorgaanisms and 0.1 to 50 mg of carotenoids, which can be supplied as a supplement with (e.g. on top of) the can.

## Claims

1. An ingestable composition for use in the protection of the skin of pets against solar radiations and the attenuation or prevention of all related skin disorders of pets which comprises at least one probiotic lactic acid bacterium or a culture supernatant thereof and at least one yeast.

2. Use of a composition comprising at least one probiotic lactic acid bacterium or a culture supernatant thereof and at least one yeast in the preparation of an ingestable composition for the protection of the skin of pets against solar radiations and the attenuation or prevention of all related skin disorders of pets.

3. A composition for use according to claim 1 or the use according to claim 2, in which the ingestable composition further includes a carotenoid.

4. A composition for use according to claim 1 or claim 3, or the use according to claim 2 or claim 3, in which the lactic acid bacterium is selected from Lactobacilli, Bifidobacteria and Enterococci.

5. A composition for use according to any one of claims 1, 3 or 4, or the use according to any one of claims 2 to 4, in which the lactic acid bacterium is *Lactobacillus johnsonii, Lactobacillus reuteri, Lactobacillus rhamnosus, Lactobacillus paracasei, Lactobacillus casei or Bifidobacterium bifidum, Bifidobacterium breve, Bifidobacterium longum, Bifidobacerium animalis, Bifidobacterium lactis, Bifidobacterium infantis, Bifidobacterium adolescentis, Bifidobacterium pseudocatenulatum, Enterococcus faecium, Enterococcus sp.* or a mixture thereof.

6. A composition for use according to any one of claims 1 or 3 to 5, or the use according to any one of claims 2 to 5, in which the lactic acid bacterium is CNCM I-1255, CNCM 1-2116, CNCM 1-2168 or CNCM 1-2170 or ATCC 27536.

7. A composition for use according to any one of claims 1 or 3 to 6, or the use according to any one of claims 2 to 6, in which the probiotic lactic acid bacterium is included into the carrier in a live form, semi-active or in desactivated form, preferably as a lyophilized powder attention.

8. A composition for use according to any one of claims 1 or 3 to 7, or the use according to any one of claims 2 to 7, wherein the carotenoid is a carotenoid with or without provitamin A activity, such as β-carotène, γ-carotene, α-carotene, lycopene, zeaxanthine and luteine, or a mixture thereof.

9. A composition for use according to any one of claims 1 or 3 to 8, or the use according to any one of claims 2 to 8, wherein the yeast is selected from the group consisting of *Dabaryomyces, Kluyveromyces, Sacharomyces, Yarrowia, Zygosaccharomyces, Candida and Rhodutorula,* or a mixture thereof.

10. A composition for use, or the use, according to claim 9, wherein the yeast is *S*. *cerevissae*

11. A composition for use according to any one of claims 1 or 3 to 10, or the use according to any one of claims 2 to 10, wherein the composition is in the form of
i) a nutritionally complete pet food in a powdered, dried or a wet, chilled or shelf stable form or,
ii) in the form of a dietary adjunct or a supplement or a treat.

12. The composition according to any one of claims 1 or 3 to 11 or the use according to any one of claims 2 to 11, wherein the probiotic lactic acid bacterium is present in an amount of from about 10⁵ to 10¹⁵ cfu/ g of the barrier.

13. The composition according to any one of claims 1 or 3 to 12 or the use according to any one of claims 2 to 12, wherein the carotenoid is present in the carrier in an amount of from 10⁻¹²% to 20% by weight.

14. The composition according to any one of claims 1 or 3 to 13 for use in reducing the effects of photoageing in pets.

15. The use according to any one of claims 2 to 13 for the preparation of a composition intended for reducing the effects of photoageing in pets.

## Patentansprüche

1. Einnehmbare Zusammensetzung für die Verwendung zum Schutz der Haut von Haustieren gegen Sonneneinstrahlung und zur Abschwächung oder Vorbeugung aller damit zusammenhängenden Hauterkrankungen von Haustieren, welche mindestens ein probiotisches Milchsäurebakterium oder einen Kulturüberstand von diesem sowie mindestens eine Hefe enthält.

2. Verwendung einer Zusammensetzung mit mindestens einem probiotischen Milchsäurebakterium oder einem Kulturüberstand von diesem und mindestens einer Hefe bei der Zubereitung einer einnehmbaren Zusammensetzung zum Schutz der Haut von Haustieren gegen Sonneneinstrahlung und zur Abschwächung oder Vorbeugung aller damit zusammenhängenden Hauterkrankungen von Haustieren.

3. Zusammensetzung für die Verwendung gemäß Anspruch 1 oder die Verwendung gemäß Anspruch 2, bei der die einnehmbare Zusammensetzung weiterhin ein Carotinoid aufweist.

4. Zusammensetzung für die Verwendung gemäß Anspruch 1 oder Anspruch 3, oder die Verwendung gemäß Anspruch 2 oder Anspruch 3, bei der das Milchsäurebakterium aus Lactobacilli, Bifidobacteria und Enterococci ausgewählt ist.

5. Zusammensetzung für die Verwendung gemäß einem der Ansprüche 1, 3 oder 4, oder die Verwendung gemäß einem der Ansprüche 2 bis 4, bei der das Milchsäurebakterium aus *Lactobacillus johnsonii, Lactobacillus reuteri, Lactobacillus rhamnosus, Lactobacillus paracasei, Lactobacillus casei oder Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum, Enterococcus faecium, Enterococcus sp.* oder einer Mischung aus diesen besteht.

6. Zusammensetzung für die Verwendung gemäß einem der Ansprüche 1 oder 3 bis 5, oder die Verwendung gemäß einem der Ansprüche 2 bis 5, bei der das Milchsäurebakterium aus CNCM I-1255, CNCM I-2116, CNCM I-2168 oder CNCM I-2170 oder ATCC 27536 besteht.

7. Zusammensetzung für die Verwendung gemäß einem der Ansprüche 1 oder 3 bis 6, oder die Verwendung gemäß einem der Ansprüche 2 bis 6, bei der das probiotische Milchsäurebakterium in den Träger in lebender Form, semiaktiver oder in deaktivierter Form, vorzugsweise als gefriergetrocknetes Pulver eingeschlossen ist.

8. Zusammensetzung für die Verwendung gemäß einem der Ansprüche 1 oder 3 bis 7, oder die Verwendung gemäß einem der Ansprüche 2 bis 7, wobei das Carotinoid ein Carotinoid mit oder ohne Provitamin A Aktivität, wie z.B. β-carotin, γ-Carotin, α-Carotin, Lykopin, Zeaxanthin und Lutein, oder eine Mischung aus diesen darstellt.

9. Zusammensetzung für die Verwendung gemäß einem der Ansprüche 1 oder 3 bis 8, oder die Verwendung gemäß einem der Ansprüche 2 bis 8, wobei die Hefe aus der Gruppe bestehend aus *Dabaryomyces, Kluyveromyces, Saccharomyces, Yarrowia, Zygosaccharomyces, Candida* und *Rhodutorula* oder einer Mischung aus diesen ausgewählt ist.

10. Zusammensetzung für die Verwendung, oder Verwendung, gemäß Anspruch 9, wobei die Hefe aus *S. cerevissae* besteht.

11. Zusammensetzung für die Verwendung gemäß einem der Ansprüche 1 oder 3 bis 10, oder Verwendung gemäß einem der Ansprüche 2 bis 10, wobei die Zusammensetzung in Form
i) einer ernährungstechnisch vollständigen Tiernahrung in Pulverform, getrockneter Form oder einer nassen, gekühlten oder haltbaren Form, oder
ii) in Form eines Nahrungszusatzes oder Ergänzungsmittels oder eines Leckerlis vorliegt.

12. Zusammensetzung gemäß einem der Ansprüche 1 oder 3 bis 11 oder Verwendung gemäß einem der Ansprüche 2 bis 11, wobei das probiotische Milchsäurebakterium in einer Menge von 10⁵ bis 10¹⁵ cfu/g des Trägers vorliegt.

13. Zusammensetzung gemäß einem der Ansprüche 1 oder 3 bis 12 oder Verwendung gemäß einem der Ansprüche 2 bis 12, wobei das Carotinoid in dem Träger in einer Menge von 10⁻¹² bis 20 Gew-% vorliegt.

14. Zusammensetzung gemäß einem der Ansprüche 1 oder 3 bis 13 für die Verwendung zur Verringerung der Auswirkungen von Lichtalterung bei Haustieren.

15. Verwendung gemäß einem der Ansprüche 2 bis 13 für die Zubereitung einer Zusammensetzung, die zur Verringerung der Auswirkungen von Lichtalterung bei Haustieren vorgesehen ist.

## Revendications

1. Composition ingérable pour une utilisation dans la protection de la peau d'animaux de compagnie contre les radiations solaires et dans l'atténuation ou la prévention de tous les troubles liés de la peau d'animaux de compagnie, qui comprend au moins une bactérie lactique probiotique ou un surnageant de culture de celle-ci et au moins une levure.

2. Utilisation d'une composition comprenant au moins une bactérie lactique probiotique ou un surnageant de culture de celle-ci et au moins une levure dans la préparation d'une composition ingérable pour la protection de la peau d'animaux de compagnie contre les radiations solaires et pour l'atténuation ou la prévention de tous les troubles liés de la peau d'animaux de compagnie.

3. Composition pour une utilisation selon la revendication 1 ou utilisation selon la revendication 2, dans laquelle la composition ingérable comprend en outre un caroténoïde.

4. Composition pour une utilisation selon la revendication 1 ou la revendication 3, ou utilisation selon la revendication 2 ou la revendication 3, dans laquelle la bactérie lactique est choisie parmi Lactobacilli, Bifidobacteria et Enterococci.

5. Composition pour une utilisation selon l'une quelconque des revendications 1, 3 ou 4, ou utilisation selon l'une quelconque des revendications 2 à 4, dans laquelle la bactérie lactique est *Lactobacillus johnsonii, Lactobacillus reuteri, Lactobacillus rhamnosus, Lactobacillus paracasei, Lactobacillus casei or Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum, Enterococcus faecium, Enterococcus sp.* ou un mélange de ceux-ci.

6. Composition pour une utilisation selon l'une quelconque des revendications 1 ou 3 à 5, ou utilisation selon l'une quelconque des revendications 2 à 5, dans laquelle la bactérie lactique est CNCM I-1255, CNCM I-2116, CNCM I-2168 ou CNCM I-2170 ou ATCC 27536.

7. Composition pour une utilisation selon l'une quelconque des revendications 1 ou 3 à 6, ou utilisation selon l'une quelconque des revendications 2 à 6, dans laquelle la bactérie lactique probiotique est incluse dans l'excipient sous forme vivante, semi-active ou sous forme désactivée, de préférence en tant que poudre lyophilisée.

8. Composition pour une utilisation selon l'une quelconque des revendications 1 ou 3 à 7, ou utilisation selon l'une quelconque des revendications 2 à 7, dans laquelle le caroténoïde est un caroténoïde avec ou sans activité de provitamine A, comme par exemple β-carotène, γ-carotène, α-carotène, lycopène, zéaxanthine et lutéine, ou un mélange de ceux-ci.

9. Composition pour une utilisation selon l'une quelconque des revendications 1 ou 3 à 8, ou utilisation selon l'une quelconque des revendications 2 à 8, dans laquelle la levure est choisie parmi le groupe constitué de *Debaryomyces, Kluyveromyces, Saccharomyces, Yarrowia, Zygosaccharomyces, Candida et Rhodutorula,* ou un mélange de ceux-ci.

10. Composition pour une utilisation, ou utilisation, selon la revendication 9, dans laquelle la levure est *S*. *cerevissae.*

11. Composition pour une utilisation selon l'une quelconque des revendications 1 ou 3 à 10, ou utilisation selon l'une quelconque des revendications 2 à 10, dans laquelle la composition est de la forme
i) d'un aliment pour animal de compagnie nutritionnellement complet sous forme poudreuse, séchée ou sous forme humide, réfrigérée ou de longue conservation ou,
ii) de la forme d'un complément alimentaire ou d'un supplément ou d'un traitement.

12. Composition selon l'une quelconque des revendications 1 ou 3 à 11, ou utilisation selon l'une quelconque des revendications 2 à 11, dans laquelle la bactérie lactique probiotique est présente dans une quantité d'environ 10⁵ à 10¹⁵ ufc/g de l'excipient.

13. Composition selon l'une quelconque des revendications 1 ou 3 à 12, ou utilisation selon l'une quelconque des revendications 2 à 12, dans laquelle le caroténoïde est présent dans l'excipient dans une quantité de 10⁻¹² % à 20 % en poids.

14. Composition selon l'une quelconque des revendications 1 ou 3 à 13 pour une utilisation dans la réduction des effets du photo-vieillissement chez les animaux de compagnie.

15. Utilisation selon l'une quelconque des revendications 2 à 13 pour la préparation d'une composition destinée à réduire les effets du photo-vieillissement chez les animaux de compagnie.
